(19) Europäisches Patentamt
European Patent Office
Office européen des brevets

(11) Veröffentlichungsnummer : **0 115 808**
**B1**

(12) # EUROPÄISCHE PATENTSCHRIFT

(45) Veröffentlichungstag der Patentschrift : **20.08.86**

(21) Anmeldenummer : **84100685.1**

(22) Anmeldetag : **24.01.84**

(51) Int. Cl.⁴ : **C 07 D335/02**, C 07 D333/48, C 07 D339/08, A 01 N 43/32

(54) **Cyclohexan-1,3-dionderivate, Verfahren zu ihrer Herstellung und ihre Verwendung zur Bekämpfung unerwünschten Pflanzenwuchses.**

(30) Priorität : **01.02.83 DE 3303182**

(43) Veröffentlichungstag der Anmeldung : **15.08.84 Patentblatt 84/33**

(45) Bekanntmachung des Hinweises auf die Patenterteilung : **20.08.86 Patentblatt 86/34**

(84) Benannte Vertragsstaaten : **AT BE CH DE FR GB IT LI NL**

(56) Entgegenhaltungen :
**EP-A- 0 070 370**
**EP-A- 0 071 707**
**Die Akte enthält technische Angaben, die nach dem Eingang der Anmeldung eingereicht wurden und die nicht in dieser Patentschrift enthalten sind.**

(73) Patentinhaber : **BASF Aktiengesellschaft**
**Carl-Bosch-Strasse 38**
**D-6700 Ludwigshafen (DE)**

(72) Erfinder : **Jahn, Dieter, Dr.**
**Burgunder Weg 8**
**D-6803 Edingen-Neckarhausen (DE)**
Erfinder : **Becker, Rainer, Dr.**
**Im Haseneck 22**
**D-6702 Duerkheim (DE)**
Erfinder : **Keil, Michael, Dr.**
**Fontanestrasse 4**
**D-6713 Freinsheim (DE)**
Erfinder : **Wuerzer, Bruno, Dr. Dipl.-Landwirt**
**Ruedigerstrasse 13**
**D-6701 Otterstadt (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue St-Denis, 75001 Paris, France

**Beschreibung**

Die Erfindung betrifft Cyclohexan-1,3-dionderivate, Verfahren zu ihrer Herstellung sowie Herbizide, die diese Verbindungen als Wirkstoff enthalten.

Es ist bekannt, Cyclohexan-1,3-dionderivate, die z. B. einen nichtaromatischen Heterocyclus als Substituenten enthalten, zur Bekämpfung von unerwünschten Gräsern in breitblättrigen Kulturen anzuwenden (DE-OS 2 439 104 und EP-A-70 370).

Es wurde gefunden, daß Cyclohexan-1,3-dionderivate der Formel

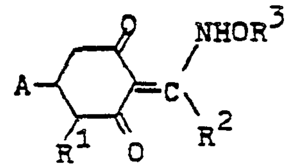

(I)

in der

A einen 4- bis 7-gliedrigen, gegebenenfalls ungesättigten Ring, der 1 oder 2 Sulfinyl- oder Sulfonylgruppen enthält und gegebenenfalls durch maximal 3 Alkylgruppen mit jeweils bis zu 4 C-Atomen, 2 Alkenylgruppen mit jeweils bis zu 6 C-Atomen oder 3 Alkoxygruppen mit jeweils bis zu 2 C-Atomen substituiert sein kann,

$R^1$ Wasserstoff, Methoxycarbonyl,

$R^2$ Alkyl mit 1 bis 4 C-Atomen und

$R^3$ Alkyl mit 1 bis 4 C-Atomen, Alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen gegen Gräser herbizid wirksam und sowohl in breitblättrigen Kulturpflanzen als auch in monokotylen Kulturen, welche nicht zur Familie der Gräser (Gramineen) zählen, selektiv sind.

Die Verbindungen der Formel I können in mehreren Formen auftreten, die alle vom Patentanspruch umfaßt werden :

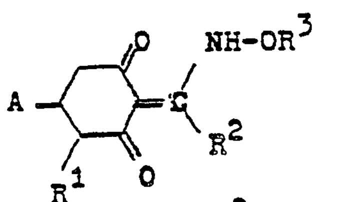

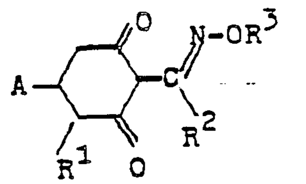

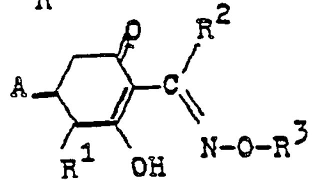

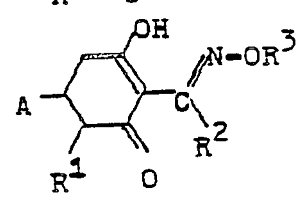

A in Formel I bedeutet einen 4- bis 7-gliedrigen, vorzugsweise 5- oder 6-gliedrigen, gesättigten oder ungesättigten Ring, der 1 oder 2 Sulfinyl- oder Sulfonylgruppen enthält. Dieser Ring kann gegebenenfalls durch maximal 3 Alkylgruppen mit jeweils bis zu 4 Kohlenstoffatomen, 2 Alkenylgruppen mit jeweils bis zu 6 Kohlenstoffatomen oder 3 Alkoxygruppen mit jeweils bis zu 2 Kohlenstoffatomen substituiert sein, wobei die Substituenten gleich oder verschieden sind. Beispiele für A sind :

1-Oxo-tetrahydrothiopyranylreste, 1,1-Dioxo-tetrahydrothiopyranylreste, 5,6-Dihydro-2H-1-oxo-thiopyranylreste, 5,6-Dihydro-2H-1,1-dioxothiopyranylreste, 1-Oxo-tetrahydrothienylreste, 1,1-Dioxo-tetrahydrothienylreste, 5,6-Dihydro-1-oxo-thienylreste oder 5,6-Dihydro-1,1-dioxothienylreste, die durch Alkylgruppen mit bis zu 4 C-Atomen, beispielsweise durch Methyl, Ethyl oder i-Propyl, vorzugsweise durch Methyl, durch Alkenylgruppen mit bis zu 6 C-Atomen, beispielsweise durch Allyl, But-2-enyl, 3-Methyl-but-2-enyl, oder durch Alkoxygruppen mit bis zu 2 C-Atomen, i. e. durch Methoxy oder Ethoxy, substituiert sind, z. B. 1-Oxo-tetrahydrothiopyran-3-yl, 1,1-Dioxo-tetrahydrothiopyran-3-yl, 5,6-Dihydro-2H-1-oxo-thiopyran-3-yl, 5,6-Dihydro-2H-1,1-dioxo-thiopyran-3-yl, 2,6-Dimethyl-1-oxo-tetrahydrothiopyran-3-yl, 2,6-Dimethyl-1,1-dioxo-tetrahydrothiopyran-3-yl, 2,6-Dimethyl-5,6-dihydro-2H-1-oxo-thiopyran-3-yl, 2,6-Dimethyl-5,6-dihydro-2H-1,1-dioxo-thiopyran-3-yl, 6-Methyl-1-oxo-tetrahydropyran-3-yl, 6-Methyl-1,1-dioxo-tetrahydropyran-3-yl, 6-Methyl-5,6-dihydro-2H-1-oxo-thiopyran-3-yl, 6-Methyl-5,6-dihydro-2H-1,1-dioxo-thiopyran-3-yl, 1-Oxo-tetrahydrothien-3-yl, 1,1-Dioxo-tetrahydrothien-3-yl, 2,5-Dihydro-1-oxo-thien-3-yl, 2,5-Dihydro-1,1-dioxo-thien-3-yl, 2-Methyl-1-oxo-tetrahydrothien-3-yl, 2-Methyl-1,1-dioxo-tetrahydrothien

-3-yl, 2,5-Dihydro-2-methyl-1-oxo-thien-3-yl, 2,5-Dihydro-2-methyl-1,1-dioxo-thien-3-yl, 2,2-Dimethyl-1-oxo-tetrahydrothien-3-yl, 2,2-Dimethyl-1,1-dioxo-tetrahydrothien-3-yl, 2,5-Dihydro-2,2-dimethyl-1-oxo-thien-3-yl, 2,5-Dihydro-2,2,-dimethyl-1,1-dioxo-thien-3-yl, 2-Methyl-2-(3-methyl-2-buten-1-yl)-1-oxo-tetrahydrothien-3-yl, 1,1-Dioxo-2-methyl-2-(3-methyl-2-buten-1-yl)-tetrahydrothien-3-yl, 2,5-Dihydro-2-methyl-2-(3-methyl-2-buten-1-yl)-thien-3-yl, 2,5-Dihydro-1,1-dioxo-2-methyl-2-(3-methyl-2-buten-1-yl)-thien-3-yl, 4-Methyl-1-oxo-tetrahydrothien-2-yl, 1,1-Dioxo-4-methyl-tetrahydrothien-2-yl, 2,5-Dihydro-4-methyl-1-oxo-thien-2-yl, 2,5-Dihydro-1,1-dioxo-4-methyl-thien-2-yl, 1-Oxo-tetrahydrothien-2-yl, 1,1-Dioxo-tetrahydrothien-2-yl, 2-Methyl-1-oxo-1,3-dithiolan-2-yl, 1,3-Dioxo-2-methyl-1,3-dithiolan-2-yl, 2-Methyl-1,1,3-trioxo-1,3-dithiolan-2-yl, 2-Methyl-1,1,3,3-tetraoxo-1,3-dithiolan-2-yl.

$R^2$ in Formel I steht für unverzweigte oder verzweigte Alkylreste mit 1 bis 4 Kohlenstoffatomen, d. h. für Methyl, Ethyl, n-Propyl, i-Propyl, n-Butyl, sec.-Butyl, i-Butyl, tert.-Butyl.

Reste für $R^3$ in Formel I sind Propargyl, Alkyl mit 1 bis 4 C-Atomen, Alkenyl mit 3 oder 4 C-Atomen oder Halogenalkenyl mit 3 oder 4 C-Atomen, das bis zu drei Halogensubstituenten enthalten kann, beispielsweise Methyl, Ethyl, n-Propyl, i-Propyl, n-Butyl, sec.-Butyl, i-Butyl, tert.-Butyl, Allyl, 1-Chlor-prop-1-en-3-yl, 2-Chlor-prop-1-en-3-yl, 1,3-Dichlor-prop-1-en-3-yl, 1,1,2-Trichlor-prop-1-en-3-yl.

Bevorzugte Cyclohexan-1,3-dionderivate sind Verbindungen der Formel I, in der $R^1$ Wasserstoff bedeutet. Weiterhin sind Verbindungen der Formel I bevorzugt, bei denen A eine 5- oder 6-gliedrigen Ring, beispielsweise einen 5,6-Dihydro-2H-1-oxo-thiopyranylrest, insbesondere einen 5,6-Dihydro-2H-1-oxo-thiopyran-3-yl-rest, einen 1-Oxo-tetrahydrothiopyranylrest, insbesondere einen 1-Oxo-tetrahydrothiopyran-3-ylrest, oder einen 1,1-Dioxo-tetrahydrothiopyranylrest, insbesondere einen 1,1-Dioxo-tetrahydrothipyran-3-ylrest bedeutet.

Als Salze der Verbindung der Formel I kommen beispielsweise die Alkalimetallsalze, insbesondere die Kalium- oder Natriumsalze, Erdalkalimetallsalze, insbesondere Calcium-, Magnesium- oder Bariumsalze, Mangan-, Kupfer-, Zink- oder Eisensalze sowie Ammonium-, Phosphonium-, Sulfonium- und Sulfoxoniumsalze, beispielsweise Ammoniumsalze, Tetraalkylammoniumsalze, Benzyltrialkylammoniumsalze, Trialkylsulfoniumsalze, Trialkylsulfoxoniumsalze, in Betracht.

Die Verbindungen der Formel I können durch Umsetzung von Verbindungen der Formel

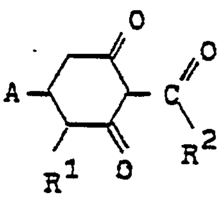

(II)

in der A, $R^1$ und $R^2$ die obengenannten Bedeutungen haben, mit Ammoniumverbindungen der Formel $R^3O—NH_3Y$, in der $R^3$ die obengenannten Bedeutungen hat und Y ein Anion bedeutet, erhalten werden.

Man führt die Reaktion zweckmäßigerweise in heterogener Phase in einem inerten Verdünnungsmittel bei einer Temperatur zwischen 0 und 80 °C oder zwischen 0 °C und dem Siedepunkt des Reaktionsgemisches in Gegenwart einer Base durch. Geeignete Basen sind beispielsweise Carbonate, Hydrogencarbonate, Acetate, Alkoholate, Hydroxide oder Oxide von Alkali- oder Erdalkalimetallen, insbesondere von Natrium, Kalium, Magnesium, Calcium. Außerdem können auch organische Basen, wie Pyridin oder tertiäre Amine, Verwendung finden.

Die Umsetzung verläuft besonders gut in einem pH-Bereich von 2 bis 9. Die Einstellung des pH-Bereiches erfolgt zweckmäßigerweise durch Zusatz von Acetaten, beispielsweise Alkalimetallacetaten, insbesondere von Natrium- oder Kaliumacetat oder einer Mischung aus beiden Salzen. Alkalimetallacetate werden beispielsweise in Mengen von 0,5 bis 2 Mol, bezogen auf die Ammoniumverbindung der Formel $R^3O—NH_3Y$, zugesetzt.

Als Lösungsmittel sind beispielsweise Dimethylsulfoxid, Alkohole, wie Methanol, Ethanol, Isopropanol, Benzol, Toluol, gegebenenfalls chlorierte Kohlenwasserstoffe, wie Chloroform, Dichlorethan, Hexan, Cyclohexan, Ester, wie Essigsäureethylester, Ether, wie Dioxan, Tetrahydrofuran, geeignet.

Die Umsetzung ist nach wenigen Stunden beendet, das Reaktionsprodukt kann dann durch Einengen der Mischung, Zugabe von Wasser und Extraktion mit einem unpolaren Lösungsmittel, wie Methylenchlorid, und Abdestillieren des Lösungsmittels unter vermindertem Druck isoliert werden.

Die Verbindungen der Formel I können außerdem durch Umsetzen der Verbindungen der Formel II mit Hydroxylaminen der Formel $R^3O—NH_2$, in der $R^3$ die obengenannten Bedeutungen hat, in inerten Verdünnungsmitteln bei einer Temperatur zwischen 0 °C und dem Siedepunkt des Reaktionsgemisches, insbesondere zwischen 15 und 70 °C, erhalten werden. Gegebenenfalls kann das Hydroxylamin in Form einer wäßrigen Lösung eingesetzt werden.

Geeignete Lösungsmittel für diese Umsetzung sind beispielsweise Alkohole, wie Methanol, Ethanol, Isopropanol, Cyclohexanol, gegebenenfalls chlorierte Kohlenwasserstoffe, wie Hexan, Cyclohexan, Methylenchlorid, Toluol, Dichlorethan, Ester, wie Essigsäureethylester, Nitrile, wie Acetonitril, cyclische Ether, wie Tetrahydrofuran.

Die Alkalimetallsalze der Verbindungen der Formel I können durch Behandeln dieser Verbindungen

mit Natrium- oder Kaliumhydroxid in wäßriger Lösung oder in einem organischen Lösungsmittel, wie Methanol, Ethanol, Aceton, Toluol, erhalten werden. Anstelle der Hydroxide können auch Natrium- und Kaliumalkoholate zur Herstellung der Alkalimetallsalze dienen.

Die übrigen Metallsalze, z. B. die Mangan-, Kupfer-, Zink-, Eisen-, Calcium-, Magnesium- und Bariumsalze können aus den Natriumsalzen durch Reaktion mit den entsprechenden Metallchloriden in wäßriger Lösung hergestellt werden. Ammonium-, Phosphonium-, Sulfonium- und Sulfoxoniumsalze können durch Umsetzung von Verbindungen der Formel I mit Ammonium-, Phosphonium-, Sulfonium- oder Sulfoxoniumhydroxiden gegebenenfalls in wäßriger Lösung erhalten werden.

Die Verbindungen der Formel II können aus Cyclohexan-1,3-dionen der Formel III, die auch in den tautomeren Formeln IIIa und IIIb vorliegen können,

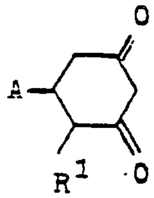
(III)

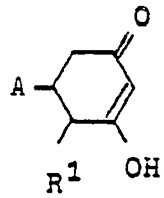
(IIIa)

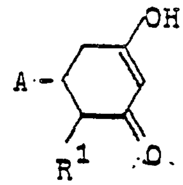
(IIIb)

nach literaturbekannten Methoden (Tetrahedron Letters, *29*, 2 491 (1975)) hergestellt werden.

Es ist auch möglich, Verbindungen der Formel II über die Zwischenstufe der Enolester, die bei der Umsetzung von Verbindungen der Formel II eventuell als Isomerengemische anfallen und in Gegenwart von Imidazol- oder Pyridinderivaten umgelagert werden (JP-OS 79/063 052), herzustellen.

Zu den Verbindungen der Formel III gelangt man nach literaturbekannten Verfahren, wie dies auf folgendem Schema hervorgeht :

(Siehe Schema Seite 5 f.)

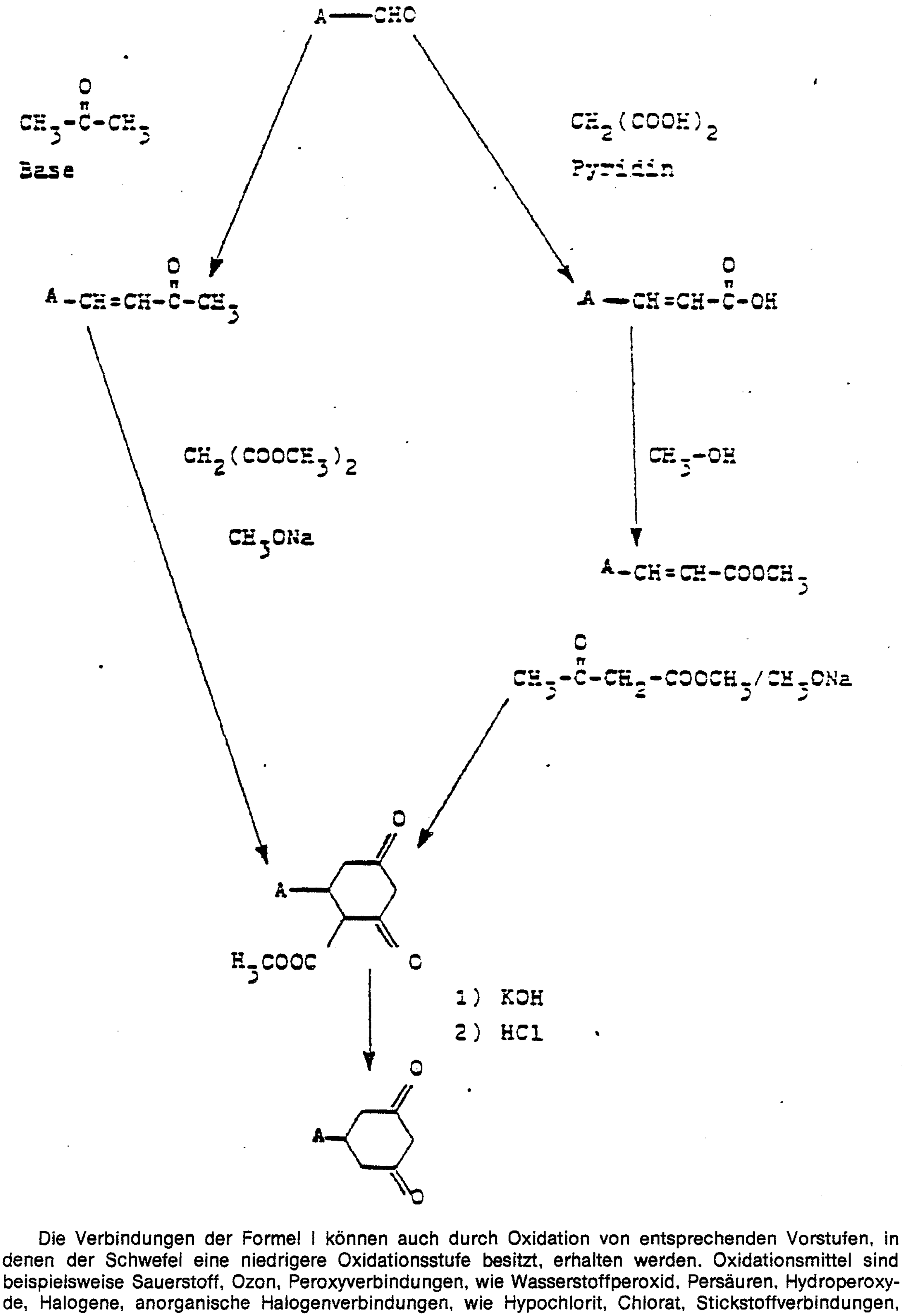


Die Verbindungen der Formel I können auch durch Oxidation von entsprechenden Vorstufen, in denen der Schwefel eine niedrigere Oxidationsstufe besitzt, erhalten werden. Oxidationsmittel sind beispielsweise Sauerstoff, Ozon, Peroxyverbindungen, wie Wasserstoffperoxid, Persäuren, Hydroperoxyde, Halogene, anorganische Halogenverbindungen, wie Hypochlorit, Chlorat, Stickstoffverbindungen, wie Salpetersäure und Distickstoffpentoxid, Salze von Metallen höherer Wertigkeit, wie Blei-, Wismut-, Vanadin-, Mangan-, Chrom-, Kobaltsalze. Auch die anodische Oxidation ist möglich. Die Oxidation kann nicht nur auf der Stufe der Oximether durchgeführt werden, sondern ist prinzipiell auf jeder Stufe des vorstehend beschriebenen Syntheseweges möglich.

Die folgenden Beispiele erläutern die Herstellung der Cyclohexan-1,3-dion-derivate der Formel I. In den Beispielen verhalten sich Gewichtsteile zu Volumenteilen wie Kilogramm zu Liter.

Beispiel 1

4,7 Gew.-Teile 2-Butyryl-5-(5,6-dihydro-2H-1,1-dioxo-thiopyran-3-yl)-cyclohexan-1,3-dion, 1,6 Gew.-Teile Ethoxyammoniumchlorid, 1,4 Gew.-Teile Natriumhydrogencarbonat und 50 Vol.-Teile Methanol werden bei Raumtemperatur 16 Stunden gerührt. Das Lösungsmittel wird unter vermindertem Druck abdestilliert, zum Rückstand werden jeweils 50 Vol.-Teile Wasser und Dichlormethan gegeben, nach dem Rühren werden die Phasen getrennt, und die wäßrige Phase wird mit 30 Vol.-Teilen Dichlormethan extrahiert. Die vereinigten organischen Phasen werden mit 1 n Salzsäure und Wasser gewaschen, über Natriumsulfat getrocknet, und das Lösungsmittel wird unter vermindertem Druck abdestilliert. Man erhält 2-(Ethoxyamino-butyliden)-5-(5,6-dihydro-2H-1,1-dioxothiopyran-3-yl)-cyclohexan-1,3-dion vom Schmp. 147 bis 149 °C (Wirkstoff Nr. 1).

Beispiel 2

11,9 Gewichtsteile 2-(Ethoxyaminobutyliden)-5-(5,6-dihydro-2H-thiopyran-3-yl)-cyclohexan-1,3-dion werden in 200 Volumentilen Chloroform gelöst. Zu dieser Lösung wird bei 5 °C eine Lösung von 14,9 Gewichtsteilen 85 %iger m-Chlorbenzoesäure zugetropft, wobei sich allmählich ein Niederschlag bildet. Die Reaktionsmischung wird innerhalb 2 Stunden auf Raumtemperatur erwärmt, der Niederschlag wird abgesaugt, die Lösung mit halbgesättigter Natriumhydrogencarbonat-Lösung extrahiert, über Natriumsulfat getrocknet und das Lösungsmittel unter vermindertem Druck abdestilliert. Man erhält ein Öl, das allmählich erstarrt. Der zerkleinerte Feststoff wird mit 100 Volumenteilen Methyl-t.-butylether bei 50 °C gerührt, abgesaugt und unter vermindertem Druck vom anhaftenden Lösungsmittel befreit. Man erhält 2-(Ethoxyaminobutyliden)-5-(5,6-dihydro-2H-1,1-dioxothiopyran-3-yl)-cyclohexan-1,3-dion als Feststoff (Wirkstoff Nr. 1) ; Schmelzpunkt : 145 bis 148 °C.

Die folgenden Verbindungen der Formel I können in gleicher Weise erhalten werden :

(Siehe Tabellen Seite 7 ff.)

| Wirkstoff Nr. | A | $R^1$ | $R^2$ | $R^3$ | $n_D$/Fp[°C] |
|---|---|---|---|---|---|
| 2 | 1,1-Dioxotetrahydrothiopyran-3-yl | H | Ethyl | Ethyl | |
| 3 | 5,6-Dihydro-2H-1,1-dioxothiopyran-3-yl | H | n-Propyl | Allyl | |
| 4 | 5,6-Dihydro-2H-1,1-dioxothiopyran-3-yl | H | Ethyl | Ethyl | |
| 5 | 5,6-Dihydro-2H-1,1-dioxothiopyran-3-yl | H | Ethyl | Allyl | |
| 6 | 5,6-Dihydro-2H-1,1-dioxothiopyran-3-yl | H | Ethyl | Propargyl | |
| 7 | 5,6-Dihydro-2H-1,1-dioxothiopyran-3-yl | H | Ethyl | 3-Chlorallyl | |
| 8 | 5,6-Dihydro-2H-1,1-dioxothiopyran-3-yl | $COOCH_3$ | Ethyl | Ethyl | |
| 9 | 5,6-Dihydro-2H-1,1-dioxothiopyran-3-yl | $COOCH_3$ | Ethyl | Allyl | |
| 10 | 5,6-Dihydro-2H-1-oxothiopyran-3-yl | H | Ethyl | Allyl | |
| 11 | 5,6-Dihydro-2H-1-oxothiopyran-3-yl | H | n-Propyl | Allyl | |
| 12 | 1-Oxotetrahydrothiopyran-3-yl | H | n-Propyl | Allyl | |
| 13 | 1-Oxotetrahydrothiopyran-3-yl | H | n-Propyl | Ethyl | |
| 14 | 1-Oxotetrahydrothiopyran-3-yl | H | Ethyl | Ethyl | |
| 15 | 1-Oxotetrahydrothiopyran-3-yl | H | Ethyl | Allyl | |
| 16 | 1,1-Dioxotetrahydrothiopyran-3-yl | H | n-Propyl | Allyl | |
| 17 | 1,1-Dioxotetrahydrothiopyran-3-yl | H | n-Propyl | Ethyl | |
| 18 | 2,6-Dimethyl-1-oxotetrahydrothiopyran-3-yl | H | n-Propyl | Ethyl | |
| 19 | 2,6-Dimethyl-1-oxotetrahydrothiopyran-3-yl | H | n-Propyl | Allyl | |
| 20 | 2,6-Dimethyl-1,1-dioxotetrahydrothiopyran-3-yl | H | n-Propyl | Ethyl | |
| 21 | 2,6-Dimethyl-1,1-dioxotetrahydrothiopyran-3-yl | H | n-Propyl | Allyl | |
| 22 | 2,6-Dimethyl-5,6-dihydro-2H-1-oxo-thiopyran--3-yl | H | n-Propyl | Allyl | |

| Wirkstoff Nr. | A | $R^1$ | $R^2$ | $R^3$ | $n_D$/Fp[°C] |
|---|---|---|---|---|---|
| 23 | 2,6-Dimethyl-5,6-dihydro-2H-1-oxo-thiopyran--3-yl | H | n-Propyl | Ethyl | |
| 24 | 2,6-Dimethyl-5,6-dihydro-2H-1,1-dioxothio-pyran-3-yl | H | n-Propyl | Ethyl | |
| 25 | 2,6-Dimethyl-5,6-dihydro-2H-1,1-dioxothio-pyran-3-yl | H | n-Propyl | Allyl | |
| 26 | 1-Oxotetrahydrothien-3-yl | H | n-Propyl | Allyl | |
| 27 | 1-Oxotetrahydrothien-3-yl | H | n-Propyl | Ethyl | |
| 28 | 1,1-Dioxotetrahydrothien-3-yl | H | n-Propyl | Ethyl | |
| 29 | 1,1-Dioxotetrahydrothien-3-yl | H | n-Propyl | Allyl | |
| 30 | 2,5-Dihydro-1-oxothien-3-yl | H | n-Propyl | Allyl | |
| 31 | 2,5-Dihydro-1-oxothien-3-yl | H | n-Propyl | Ethyl | |
| 32 | 2,5-Dihydro-1,1-dioxothien-3-yl | H | n-Propyl | Ethyl | |
| 33 | 2,5-Dihydro-1,1-dioxothien-3-yl | H | n-Propyl | Allyl | |
| 34 | 2-Methyl-1-oxotetrahydrothien-3-yl | H | n-Propyl | Allyl | |
| 35 | 2-Methyl-1-oxotetrahydrothien-3-yl | H | n-Propyl | Ethyl | |
| 36 | 2,5-Dihydro-2-methyl-1-oxothien-3-yl | H | n-Propyl | Ethyl | |
| 37 | 2,5-Dihydro-2-methyl-1-oxothien-3-yl | H | n-Propyl | Allyl | |
| 38 | 2,5-Dihydro-2-methyl-1,1-dioxothien-3-yl | H | n-Propyl | Allyl | |
| 39 | 2,5-Dihydro-2-methyl-1,1-dioxothien-3-yl | H | n-Propyl | Ethyl | |
| 40 | 2,2-Dimethyl-1-oxotetrahydrothien-3-yl | H | n-Propyl | Ethyl | |

| Wirkstoff Nr. | A | $R^1$ | $R^2$ | $R^3$ | $n_D$/Fp[°C] |
|---|---|---|---|---|---|
| 41 | 2,2-Dimethyl-1-oxotetrahydrothien-3-yl | H | n-Propyl | Allyl | |
| 42 | 2,2-Dimethyl-1,1-dioxotetrahydrothien-3-yl | H | n-Propyl | Allyl | |
| 43 | 2,2-Dimethyl-1,1-dioxotetrahydrothien-3-yl | H | n-Propyl | Ethyl | |
| 44 | 2,5-Dihydro-2,2-dimethyl-1-oxothien-3-yl | H | n-Propyl | Ethyl | |
| 45 | 2,5-Dihydro-2,2-dimethyl-1-oxothien-3-yl | H | n-Propyl | Allyl | |
| 46 | 2,5-Dihydro-2,2-dimethyl-1,1-dioxothien-3-yl | H | n-Propyl | Allyl | |
| 47 | 2,5-Dihydro-2,2-dimethyl-1,1-dioxothien-3-yl | H | n-Propyl | Ethyl | |
| 48 | 2-Methyl-2-(3-methyl-2-buten-1-yl)-1-oxo-tetrahydrothien-3-yl | H | n-Propyl | Ethyl | |
| 49 | 2-Methyl-2-(3-methyl-2-buten-1-yl)-1-oxo-tetrahydrothien-3-yl | H | n-Propyl | Allyl | |
| 50 | 1,1-Dioxo-2-methyl-2-(3-methyl-2-buten-1-yl)-tetrahydrothien-3-yl | H | n-Propyl | Allyl | |
| 51 | 1,1-Dioxo-2-methyl-2-(3-methyl-2-buten-1-yl)-tetrahydrothien-3-yl | H | n-Propyl | Ethyl | |
| 52 | 2,5-Dihydro-2-methyl-2-(3-methyl-2-buten-1-yl)--1-oxothien-3-yl | H | n-Propyl | Ethyl | |
| 53 | 2,5-Dihydro-2-methyl-2-(3-methyl-2-buten-1-yl)--1-oxothien-3-yl | H | n-Propyl | Allyl | |
| 54 | 2,5-Dihydro-1,1-dioxo-2-methyl-2-(3-methyl--2-buten-1-yl)-thien-3-yl | H | n-Propyl | Ethyl | |
| 55 | 2,5-Dihydro-1,1-dioxo-2-methyl-2-(3-methyl--2-buten-1-yl)-thien-3-yl | H | n-Propyl | Allyl | |

| Wirkstoff Nr. | A | $R^1$ | $R^2$ | $R^3$ | $n_D$/Fp[°C] |
|---|---|---|---|---|---|
| 56 | 4-Methyl-1-oxotetrahydrothien-2-yl | H | n-Propyl | Allyl | |
| 57 | 4-Methyl-1-oxotetrahydrothien-2-yl | H | n-Propyl | Ethyl | |
| 58 | 1,1-Dioxo-4-methyltetrahydrothien-2-yl | H | n-Propyl | Ethyl | |
| 59 | 1,1-Dioxo-4-methyltetrahydrothien-2-yl | H | n-Propyl | Allyl | |
| 60 | 2,5-Dihydro-1,1-dioxo-4-methylthien-2-yl | H | n-Propyl | Allyl | |
| 61 | 2,5-Dihydro-1,1-dioxo-4-methylthien-2-yl | H | n-Propyl | Ethyl | |
| 62 | 2,5-Dihydro-4-methyl-1-oxo-thien-2-yl | H | n-Propyl | Ethyl | |
| 63 | 2,5-Dihydro-4-methyl-1-oxo-thien-2-yl | H | n-Propyl | Allyl | |
| 64 | 1-Oxotetrahydrothien-2-yl | H | n-Propyl | Allyl | |
| 65 | 1-Oxotetrahydrothien-2-yl | H | n-Propyl | Ethyl | |
| 66 | 1,1-Dioxotetrahydrothien-2-yl | H | n-Propyl | Ethyl | |
| 67 | 1,1-Dioxotetrahydrothien-2-yl | H | n-Propyl | Allyl | |
| 68 | 2-Methyl-1-oxo-1,3-dithiolan-2-yl | H | n-Propyl | Allyl | |
| 69 | 2-Methyl-1-oxo-1,3-dithiolan-2-yl | H | n-Propyl | Ethyl | |
| 70 | 1,3-Dioxo-2-methyl-1,3-dithiolan-2-yl | H | n-Propyl | Ethyl | |
| 71 | 1,3-Dioxo-2-methyl-1,3-dithiolan-2-yl | H | n-Propyl | Allyl | |
| 72 | 2-Methyl-1,1,3-trioxo-1,3-dithiolan-2-yl | H | n-Propyl | Allyl | |
| 73 | 2-Methyl-1,1,3-trioxo-1,3-dithiolan-2-yl | H | n-Propyl | Ethyl | |

| Wirkstoff Nr. | A | $R^1$ | $R^2$ | $R^3$ | $n_D$/Fp[°C] |
|---|---|---|---|---|---|
| 74 | 2-Methyl-1,1,3,3-tetraoxo-1,3-dithiolan-2-yl | H | n-Propyl | Ethyl | |
| 75 | 2-Methyl-1,1,3,3-tetraoxo-1,3-dithiolan-2-yl | H | n-Propyl | Allyl | |
| 76 | 1-Oxotetrahydrothiopyran-3-yl | H | n-Propyl | 3-Chlorallyl | |
| 77 | 1-Oxotetrahydrothiopyran-3-yl | H | n-Propyl | Propargyl | |
| 78 | 1-Oxotetrahydrothiopyran-3-yl | H | Ethyl | Propargyl | |
| 79 | 1-Oxotetrahydrothiopyran-3-yl | H | Ethyl | 3-Chlorallyl | |
| 80 | 1-Oxotetrahydrothiopyran-3-yl | H | Methyl | Allyl | |
| 81 | 1-Oxotetrahydrothiopyran-3-yl | H | Methyl | Ethyl | |
| 82 | 1,1-Dioxotetrahydrothiopyran-3-yl | H | n-Propyl | Propargyl | |
| 83 | 1,1-Dioxotetrahydrothiopyran-3-yl | H | n-Propyl | 3-Chlorallyl | |
| 84 | 1,1-Dioxotetrahydrothiopyran-3-yl | H | Ethyl | 3-Chlorallyl | |
| 85 | 1,1-Dioxotetrahydrothiopyran-3-yl | H | Ethyl | Propargyl | |
| 86 | 1,1-Dioxotetrahydrothiopyran-3-yl | H | Methyl | Ethyl | |
| 87 | 1,1-Dioxotetrahydrothiopyran-3-yl | H | Methyl | Allyl | |
| 88 | 5,6-Dihydro-2H-1-oxothiopyran-3-yl | H | n-Propyl | Ethyl | |
| 89 | 5,6-Dihydro-2H-1-oxothiopyran-3-yl | H | n-Propyl | Propargyl | |
| 90 | 5,6-Dihydro-2H-1-oxothiopyran-3-yl | H | n-Propyl | 3-Chlorallyl | |
| 91 | 5,6-Dihydro-2H-1-oxothiopyran-3-yl | H | Ethyl | n-Propyl | |
| 92 | 5,6-Dihydro-2H-1-oxothiopyran-3-yl | H | Methyl | Ethyl | |

| Wirkstoff Nr. | A | $R^1$ | $R^2$ | $R^3$ | $n_D$/Fp[°C] |
|---|---|---|---|---|---|
| 93 | 5,6-Dihydro-2H-1-oxothiopyran-3-yl | H | Methylyl | Allyl | |
| 94 | 5,6-Dihydro-2H-1-oxothiopyran-3-yl | H | Ethyl | Ethyl | |
| 95 | 5,6-Dihydro-2H-1,1-dioxothiopyran-3-yl | H | n-Propyl | Propargyl | |
| 96 | 5,6-Dihydro-2H-1,1-dioxothiopyran-3-yl | H | n-Propyl | 3-Chlorallyl | |
| 97 | 5,6-Dihydro-2H-1,1-dioxothiopyran-3-yl | H | Ethyl | n-Propyl | |
| 98 | 1-Oxo-tetrahydrothiopyran-4-yl | H | n-Propyl | Ethyl | |
| 99 | 1-Oxo-tetrahydrothiopyran-4-yl | H | n-Propyl | Allyl | |
| 100 | 1,1-Dioxotetrahydrothiopyran-4-yl | H | n-Propyl | Allyl | |
| 101 | 1,1-Dioxotetrahydrothiopyran-4-yl | H | n-Propyl | Ethyl | |

Zur Identifizierung der Cyclohexan-1,3-dionderivate der Formel I dienen 1H-NMR-Daten :

| Verbindung Nr. | $^1$H-NMR-Daten [ppm], bezogen auf Tetramethylsilan als internen Standard |
|---|---|
| 1 | 1,0 (t), 3,60 (s), 5,75 (s) |
| 2 | 1,15 (t), 1,32 (t), 2,20 (m), 4,10 (q) |
| 4 | 1,15 (t), 2,60 (s), 3,60 (s), 5,75 (s) |
| 5 | 0,97 (t), 3,80 (s), 4,55 (d), 5,70 (s) |
| 8 | 1,10 (t), 3,55 (s), 3,75 (s), 4,10 (q) |
| 9 | 1,15 (t), 3,78 (s), 4,55 (d) |
| 10 | 1,20 (t), 2,60 (m), 4,58 (d), 5,75 (s) |
| 13 | 0,97 (t), 1,32 (t), 4,15 (q) |
| 14 | 1,18 (t), 1,33 (t), 4,13 (q) |
| 17 | 1,00 (t), 2,15 (s), 4,15 (q) |
| 18 | 0,82 (t), 1,16 3,98 (q) |
| 20 | 0,85 (t), 1,70 (t), 4,05 (q) |
| 80 | 2,40 (s), 4,60 (d), 6,0 (m) |
| 81 | 1,33 (t), 2,40 (s), 3,48 (m) |
| 86 | 1,35 (t), 2,40 (s), 3,05 (m) |
| 87 | 2,40 (s), 2,55 (m), 4,68 (d) |
| 88 | 1,00 (t), 1,58 (q), 2,60 (m), 5,75 (s) |
| 89 | 0,98 (t), 1,58 (q), 2,58 (s), 4,78 (d) |
| 90 | 0,95 (t), 1,56 (q), 2,68 (m), 4,54 (d) |
| 91 | 1,20 (t), 1,80 (q), 2,60 (m) |
| 94 | 1,40 (t), 2,60 (m), 3,30 (m), 4,15 (q) |
| 95 | 0,95 (t), 2,52 (s), 3,55 (s), 4,67 (d) |
| 96 | 0,98 (t), 3,58 (s), 4,55 (d), 4,80 (d) |
| 97 | 3,15 (m), 3,76 (s), 3,95 (t), 5,70 (s) |

Die Cyclohexan-1,3-dionderivate der Formel I eignen sich zur Bekämpfung von Gräsern und bewirken außerdem eine Erhöhung des Kohlehydratgehaltes in den Blättern der behandelten Pflanzen.

Die Cyclohexan-1,3-dionderivate der Formel I können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz-, und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung Nr. 1 mit 10 Gewichtsteilen N-Methyl-α-pyrrolidon und erhält eine Lösung, die zur Anwendung in Form kleinster Tropfen geeignet ist.

II. 10 Gewichtsteile der Verbindung Nr. 2 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanolamid, 2 Gewichtsteilen Calciumsalz der Dodecyl-benzolsulfonsäure und 2 Gewichtsteilen des Anlagerungsproduktes von 40 Mol Ethylenoxid an 1 Mol Ricinusöl besteht.

III. 20 Gewichtsteile der Verbindung Nr. 13 werden in einer Mischung gelöst, die aus 60 Gewichtsteilen Cyclohexanon, 30 Gewichsteilen Isobutanol, 5 Gewichtsteilen des Anlagerungsproduktes von 7 Mol Ethylenoxid an 1 Mol Isooctylphenol und 5 Gewichtsteilen des Anlagerungsproduktes von 40 Mol Ethylenoxid an 1 Mol Ricinusöl besteht.

IV. 20 Gewichtsteile der Verbindung Nr. 14 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 80 Gewichtsteile der Verbindung Nr. 88 werden mit 3 Gewichtsteilen des Natriumsalzes der Diisobutyl-naphthalin-sulfonsäure, 10 Gewichtsteilen des Natriumsalzes einer Ligninsulfonsäure aus einer Sulfit-Ablauge und 7 Gewichtsteilen pulverförmigem Kieselsäuregel gut vermischt und in einer Hammermühle vermahlen.

VI. 5 Gewichtsteile der Verbindung Nr. 17 werden mit 95 Gewichtsteilen feinteiligem Kaolin vermischt. Man erhält auf diese Weise ein Stäubemittel, das 5 Gewichtsprozent des Wirkstoffs enthält.

VII. 30 Gewichtsteile der Verbindung Nr. 88 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung Nr. 1 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation kann im Vorauflauf- oder im Nachauflaufverfahren erfolgen. Sind die Wirkstoffe bei Nachauflaufanwendung für gewisse Kulturpflanzen weniger verträglich, können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünscher Pflanzen oder die unbedeckte Bodenfläche gelangen (post-directed, lay-by).

Die Aufwandmengen an Wirkstoff betragen je nach Jahreszeit, Zielpflanzen und Wachstumsstadien, Bodenart und Anwendungsverfahren 0,025 bis 3 kg/ha, vorzugsweise 0,1 bis 1,0 kg/ha.

Die herbizide Wirkung der Cyclohexan-1,3-dionderivate der Formel I läßt sich durch Gewächshausversuche zeigen :

Als Kulturgefäße dienen Plastikblumentöpfe mit 300 $cm^3$ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Bei dieser Applikationsmethode beträgt die Aufwandmenge 0,5 kg Wirkstoff/ha. Nach

dem Aufbringen der Mittel geregnet man die Gefäße leicht, um Keimung und Wachstum in Gang zu bringen. Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull angereicherten Substrat angezogen. Es werden entweder direkt gesäte und in den gleichen Gefäßen aufgewachsene Pflanzen verwendet, oder aber solche, die erst als Keimpflanzen getrennt angezogen und einige Tage vor der Behandlung in die Versuchsgefäße verpflanzt werden. Die Aufwandmenge beträgt 0,125 kg Wirkstoff/ha. Eine Abdeckung unterbleibt bei der Nachauflaufbehandlung.

Die Versuchsgefäße werden im Gewächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen gepflegt, und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

Die in den Gewächshausversuchen verwendeten Pflanzen setzen sich aus folgenden Arten zusammen :

Echinochloa crus-galli (Hühnerhirse), Glycine max (Soja), Rottboellia exaltata, Setaria italica (Kolbenhirse), Sorghum halepense (Sudangras), Bromus inermis (unbegrannte Trespe), Digitaria sanguinalis (Blutfingerhirse), Triticum aestivum (Weizen).

Sowohl bei Vor- als auch bei Nachauflaufanwendung zeigt beispielsweise die Verbindung Nr. 1 eine gute herbizide Aktivität gegen unerwünschte Gräser. Sojabohnen als breitblättrige Beispielskultur blieb völlig ungeschädigt. Die Verbindungen Nr. 2, 13, 14, 17 und 88 zeigen bei einer Aufwandmenge von 0,125 kg Wirkstoff/ha und Nachauflaufanwendung eine sehr gute Wirkung gegen unerwünschte Grasarten ; dabei wird Soja ebenfalls nicht geschädigt.

In Anbetracht der Verträglichkeit der Applikationsmethoden können die erfindungsgemäßen Verbindungen noch in einer weiteren Zahl von Kulturpflanzen zur Beseitigung unerwünschter Wildgräser oder grasartiger Kulturpflanzen, sofern sie an gewissen Standorten unerwünscht sind, eingesetzt werden. In Betracht kommen beispielsweise folgende Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor — Färberdistel |
| Caryg illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium | Baumwolle |
| Helianthus annuus | Sonnenblume |
| Helianthus tuberosus | Topinambur |
| Hevea brasiliensis | Parakautschukbaum |

| Botanischer Name | Deutscher Name |
|---|---|
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Metha piperita | Pfefferminze |
| Musa spp. | Obst- und Mehlbanane |
| Nicotiana tabacum (N. rustica) | Tabak |
| Olea europaea | Ölbaum |
| Phaseolus lunatus | Mondbohne |
| Phaseolus mungo | Erdbohne |
| Phaseolus vulgaris | Buschbohnen |
| Petroselinum crispum spp. tuberosum | Wurzelpetersilie |
| Picea abis | Rotfichte |
| Abies alba | Weißtanne |
| Pinus spp. | Kiefer |
| Pisum sativum | Gartenerbse |
| Prunus avium | Süßkirsche |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sasamum indicum | Sesam |
| Solanum tuberosum | Kartoffel |
| Sorghum bicolor (s. vulgare) | Mohrenhirse |
| Prunus domestica | Pflaume |
| Sorghum dochna | Zuckerhirse |
| Spinacia oleracea | Spinat |
| Theobroma cacao | Kakaobaum |
| Trifolium pratense | Rotklee |
| Vaccinium corymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |

Zur Verbreiterung des Wirkungsspektrums und zur Erzielung synergistischer Effekte können die Cyclohexan-1,3-dionderivate der Formel I bzw. die sie enthaltenden Mittel auch mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Triazinone, Uracile, Benzofuranderivate, andere Cyclohexan-1,3-dion-derivate und andere herbizide Wirkstoffe in Betracht, wie

3-(1-Methylethyl)-1H-2,1,3-benzothiadiazin-4(3H)-on-2,2-dioxid und Salze
3-(1-Methylethyl)-8-chlor-1H-2,1,3-benzothiadiazin-4(3H)-on-2,2-dioxid und Salze
3-(1-Methylethyl)-8-fluor-1H-2,1,3-benzothiadiazin-4(3H)-on-2,2-dioxid und Salze
3-(1-Methylethyl)-8-methyl-1H-2,1,3-benzothiadiazin-4(3H)-on-2,2-dioxid und Salze

1-Methoxymethyl-3-(1-methylethyl)-2,1,3-benzothiadiazin-4(3H)-on-2,2-dioxid
1-Methoxymethyl-8-chlor-3-(1-methylethyl)-2,1,3-benzothiadiazin-4(3H)-on-2,2-dioxid
1-Methoxymethyl-8-fluor-3-(1-methylethyl)-2,1,3-benzothiadiazin-4(3H)-on-2,2-dioxid
1-Cyan-8-chlor-3-(1-methylethyl)-2,1,3-benzothiadiazin-4(3H)-on-2,2-dioxid

1-Cyan-8-fluor-3-(1-methylethyl)-2,1,3-benzothiadiazin-4(3H)-on-2,2-dioxid
1-Cyan-8-methyl-3-(1-methylethyl)-2,1,3-benzothiadiazin-4(3H)-on-2,2-dioxid
1-Azidomethyl-3-(1-methylethyl)-2,1,3-benzothiadiazin-4(3H)-on-2,2-dioxid
3-(1-Methylethyl)-1H-(pyridino-[3,2-e]2,1,3-thiadiazin-(4)-on-2,2-dioxid
1-Cyan-3-(1-methylethyl)-2,1,3-benzothiadiazin-4(3H)-on-2,2-dioxid
N-(1-Ethylpropyl)-2,6-dinitro-3,4-dimethylanilin
N-(1-Methylethyl)-N-ethyl-2,6-dinitro-4-trifluormethyl-anilin
N-n-Propyl-N-ß-chlorethyl-2,6-dinitro-4-trifluormethyl-anilin
N-n-Propyl-N-cyclopropylmethyl-2,6-dinitro-4-trifluor-methyl-anilin
N,N-Di-n-propyl-2,6-dinitro-3-amino-4-trifluormethylanilin
N,N-Di-n-propyl-2,6-dinitro-4-methyl-anilin
N,N-Di-n-propyl-2,6-dinitro-4-methylsulfonyl-anilin
N,N-Di-n-propyl-2,6-dinitro-4-aminosulfonyl-anilin
N,N-Di-beta-chlorethyl-2,6-dinitro-4-methyl-anilin
N-Ethyl-N-(2-methylallyl)-2,6-dinitro-4-trifluormethyl-anilin

N-Methylcarbaminsäure-3,4-dichlorbenzylester
N-Methylcarbaminsäure-2,6-di-tert.butyl-4-methylphenyl-ester
N-Phenylcarbaminsäure-isopropylester
N-3-Chlorphenylcarbaminsäure-isopropylester
N-3-Chlorphenylcarbaminsäure-butin-1-yl-3-ester
N-3-Chlorphenylcarbaminsäure-4-chlor-butin-2-yl-ester
N-3,4-Dichlorphenylcarbaminsäure-methylester
N-(4-Amino-benzosulfonyl)-carbaminsäure-methylester
O-(N-Phenylcarbamoyl)-propanonoxim
N-Ethyl-2-(phenylcarbamoyl)-oxypropionsäureamid
3'-N-Isopropyl-carbamoyloxy-propionanilid

Ethyl-N-[3-(N'-phenylcarbamoyloxy)-phenyl]-carbamat
Methyl-N-[3-(N'-methyl-N'-phenylcarbamoyloxy)-phenyl]-carbamat
Isopropyl-N-[3-(N'-ethyl-N'-phenylcarbamoyloxy)-phenyl]-carbamat
Methyl-N-[3-(N'-3-methylphenylcarbamoyloxy)-phenyl]-carbamat
Ethyl-N-[3-(N'-3-chlor-4-fluorphenylcarbamoyloxy)-phenyl]-carbamat
Ethyl-N-[3-(N'-3,4-difluorphenylcarbamoyloxy)-phenyl]-carbamat

N,N-Diethyl-thiolcarbaminsäure-p-chlorbenzylester
N,N-Di-n-propyl-thiolcarbaminsäure-ethylester
N,N-Di-n-propyl-thiolcarbaminsäure-n-propylester
N,N-Di-isopropyl-thiolcarbaminsäure-2,3-dichlorallylester
N,N-Di-isopropyl-thiolcarbaminsäure-2,3,3-trichlorallylester
N,N-Di-isopropyl-thiolcarbaminsäure-3-methyl-5-isoxazolyl-methylester
N,N-Di-isopropyl-thiolcarbaminsäure-3-ethyl-5-isoxazolyl-methylester
N,N-Di-sec.butyl-thiolcarbaminsäure-ethylester
N,N-Di-sec.butyl-thiolcarbaminsäure-benzylester
N-Ethyl-N-cyclohexyl-thiolcarbaminsäure-ethylester
N-Ethyl-N-bicyclo[2.2.1]heptyl-thiolcarbaminsäureethylester
S-Ethyl-hexahydro-1H-azepin-1-carbothiolat
S-Ethyl-3-methylhexahydro-1-H-azepin-1-carbothiolat
N-Ethyl-N-n-butyl-thiolcarbaminsäure-n-propylester
N,N-Dimethyl-dithiocarbaminsäure-2-chlorallylester
N-Methyl-dithiocarbaminsäure-Natriumsalz
Trichloressigsäure-Natriumsalz
Alpha,alpha-Dichlorpropionsäure-Natriumsalz
Alpha,alpha-Dichlorbuttersäure-Natriumsalz
Alpha,alpha,beta,beta-Tetrafluorpropionsäure-Natriumsalz
Alpha-Methyl-alpha,beta-dichlorpropionsäure-Natriumsalz
Alpha-Chlor-beta-(4-chlorphenyl)-propionsäure-methylester
Alpha,beta-Dichlor-beta-phenylpropionsäure-methylester
Benzamido-oxy-essigsäure
2,3,5-Trijodbenzoesäure (Salze, Ester, Amide)
2,3,6-Trichlorbenzoesäure (Salze, Ester, Amide)
2,3,5,6-Tetrachlorbenzoesäure (Salze, Ester, Amide)
2-Methoxy-3,6-dichlorbenzoesäure (Salze, Ester, Amide)
2-Methoxy-3,5,6-trichlorbenzoesäure (Salze, Ester, Amide)
3-Amino-2,5,6-trichlorbenzoesäure (Salze, Ester, Amide)

O,S-Dimethyl-tetrachlor-thioterephthalat
Dimethyl-2,3,5,6-tetrachlor-terephthalat
Dinatrium-3,6-endoxohexahydro-phthalat
4-Amino-3,5,6-trichlor-picolinsäure (Salze)
2-Cyan-3-(N-methyl-N-phenyl)-amino-acrylsäureethylester
2-[4-(4'-Chlorphenoxy)-phenoxy]-propionsäureisobutylester
2-[4-(2',4'-Dichlorphenoxy)-phenoxy]-propionsäuremethylester
2-[4-(4'-Trifluormethylphenoxy)-phenoxy]-propionsäure-methylester
2-[4-(2'-Chlor-4'-trifluorphenoxy)-phenoxy]-propionsäure Natriumsalz
2-[4-(3',5'-Dichlorpyridyl-2-oxy)-phenoxy]-propionsäure Natriumsalz

2-(N-Benzoyl-N-(3,4-dichlorphenyl)-amino)-propionsäureethylester
2-(N-Benzoyl-3-chlor-4-fluorphenylamino)-propionsäure-methylester
2-(N-Benzoyl-3-chlor-4-fluorphenylamino)-propionsäureisopropylester

2-Chlor-4-ethylamino-6-isopropylamino-1,3,5-triazin
2-Chlor-4-ethylamino-6-(amino-2'-propionitril)-1,3,5-triazin
2-Chlor-4,6-bisethylamino-1,3,5-triazin
2-Chlor-4,6-bisisopropylamino-1,3,5-triazin
2-Chlor-4-isopropylamino-6-cyclopropylamino-1,3,5-triazin

2-Azido-4-methylamino-6-isopropylamino-1,3,5-triazin
2-Methylthio-4-ethylamino-6-isopropylamino-1,3,5-triazin
2-Methylthio-4-ethylamino-6-tert.butylamino-1,3,5-triazin
2-Methylthio-4,6-bisethylamino-1,3,5-triazin
2-Methylthio-4,6-bisisopropylamino-1,3,5-triazin
2-Methoxy-4-ethylamino-6-isopropylamino-1,3,5-triazin
2-Methoxy-4,6-bisethylamino-1,3,5-triazin
2-Methoxy-4,6-bisisopropylamino-1,3,5-triazin
4-Amino-6-tert.butyl-3-methylthio-4,5-dihydro-1,2,4-triazin-5-on
4-Amino-6-phenyl-3-methyl-4,5-dihydro-1,2,4-triazin-5-on
4-Isobutylidenamino-6-tert.butyl-3-methylthio-4,5-dihydro-1,2,4-triazin-5-on
1-Methyl-3-cyclohexyl-6-dimethylamino-1,3,5-triazin-2,4-dion

3-tert.Butyl-5-chlor-6-methyluracil
3-Isopropyl-5-brom-6-methyluracil
3-sec.Butyl-5-brom-6-methyluracil
3-Cyclohexyl-5,6-trimethylenuracil

2-Methyl-4-(3'-trifluormethylphenyl)-tetrahydro-1,2,4-oxadiazin-3,5-dion
2-Methyl-4-(4'-fluorphenyl)-tetrahydro-1,2,4-oxadiazin-3,5-dion
3-Amino-1,2,4-triazol
1-(4-Chlorphenoxy-3,3-dimethyl-1-(H-1,2,4-triazolyl)-2-butanon
N,N-Diallylchloracetamid
N-Isopropyl-2-chloracetanilid
N-(1-Methyl-propin-2-yl)-2-chloracetanilid

2-Methyl-6-ethyl-N-ethoxymethyl-2-chloracetanilid
2-Methyl-6-ethyl-N-(2-methoxy-1-methylethyl)-2-chloracetanilid
2-Methyl-6-ethyl-N-(isopropoxycarbonylethyl)-2-chloracet-anilid
2-Methyl-6-ethyl-N-(pyrazolyl-methyl)-2-chloracetanilid
2,6-Dimethyl-N-(pyrazolyl-methyl)-2-chloracetanilid
2,6-Dimethyl-N-(4-methylpyrazolyl-methyl)-2-chloracetanilid
2,6-Dimethyl-N-(1,2,4-triazolyl-methyl)-2-chloracetanilid
2,6-Dimethyl-N-(3,5-dimethylpyrazolyl-methyl)-2-chloracetanilid
2,6-Dimethyl-N-(1,3-dioxalan-2-yl-methyl)-2-chloracetanilid
2,6-Dimethyl-N-(2-methoxyethyl)-2-chloracetanilid
2,6-Dimethyl-N-isobutoxymethyl-2-chloracetanilid
2,6-Diethyl-N-methoxymethyl-2chloracetanilid
2,6-Diethyl-N-(n-butoxymethyl)-2-chloracetanilid
2,6-Diethyl-N-ethoxycarbonylmethyl-2-chloracetanilid
2,3-Dimethyl-N-isopropyl-2-chloracetanilid
2,6-Diethyl-N-(2-n-propoxy-ethyl)-2-chloracetanilid

alpha-(2-Methyl-4-chlorphenoxy)-N-methoxy-acetamid

2-(alpha-Naphthoxy)-N,N-diethylpropionamid
2,2-Diphenyl-N,N-dimethylacetamid
alpha-(3,4,5-Tribrompyrazolyl)-N,N-dimethylpropionamid
N-(1,1-Dimethylprop-2-inyl)-3,5-dichlorbenzamid
N-Naphth-1-yl-phthalamidsäure
Propionsäure-3,4-dichloranilid
Cyclopropancarbonsäure-3,4-dichloranilid
Methacrylsäure-3,4-dichloranilid
2-Methylpentancarbonsäure-3,4-dichloranilid
5-Acetamido-2,4-dimethyltrifluormethan-sulfonanilid
5-Acetamido-4-methyl-trifluormethan-sulfonanilid

2-Propionyl-amino-4-methyl-5-chlor-thiazol
0-(Methylaminosulfonyl)-glykolsäure-hexamethylenimid
2,6-Dichlor-thiobenzamid
2,6-Dichlorbenzonitril
3,5-Dibrom-4-hydroxy-benzonitril (Salze)
3,5-Dijod-4-hydroxy-benzonitril (Salze)
3,5-Dibrom-4-hydroxy-0-2,4-dinitrophenylbenzaldoxim (Salze)
Pentachlorphenyl-Natriumsalz
2,4-Dichlorphenyl-4'-nitrophenylether
2,4,6-Trichlorphenyl-4'-nitrophenylether
2-Fluor-4,6-dichlorphenyl-4'-nitrophenylether
2-Chlor-4-trifluormethylphenyl-4'-nitrophenylether
2,4'-Dinitro-4-trifluormethyl-diphenylether
2,4-Dichlorphenyl-3'-methoxy-4'-nitro-phenylether
2-Chlor-4-trifluormethylphenyl-3'-ethoxy-4'-nitro-phenylether
2-Chlor-4-trifluormethylphenyl-3'-carboxy-4'-nitro-phenylether (Salze)
2,4-Dichlorphenyl-3'-methoxycarbonyl-4'-nitro-phenylether
2-(3,4-Dichlorphenyl)-4-methyl-1,2,4-oxadiazolidin-3,5-dion
2-(3-Isopropylcarbamoyl-oxyphenyl)-4-methyl-1,2,4-oxadiazolidin-3,5-dion
2-Phenyl-3,1-benzoxazinon-(4)
3-(4-Bromphenyl)-3,4,5,9,10-pentaazatetracyclo-[5,4,1,$0^{2,6}$,$0^{8,11}$]-dodeca-3,9-dien
2-Ethoxy-2,3-dihydro-3,3-dimethyl-5-benzofuranyl-methan-sulfonat
2-Methyl-4,6-dinitrophenol (Salze, Ester)

2-sec.Butyl-4,6-dinitrophenol (Salze, Ester)
2-sec.Butyl-4,6-dinitrophenol-acetat
2-tert.Butyl-4,6-dinitrophenol-acetat
2-tert.Butyl-4,6-dinitrophenol (Salze)
2-tert.Butyl-5-methyl-4,6-dinitrophenol (Salze)
2-tert.Butyl-5-methyl-4,6-dinitrophenol-acetat
2-sec.Amyl-4,6-dinitrophenol (Salze, Ester)
1-(alpha,alpha-Dimethylbenzyl)-3-(4-methylphenyl)-harnstoff
1-Phenyl-3-(2-methylcyclohexyl)-harnstoff
1-(4-Chlorphenyl)-3,3-dimethyl-harnstoff
1-(4-Chlorphenyl)-3-methyl-3-(butin-1-yl-3)-harnstoff
1-(3,4-Dichlorphenyl)-3,3-dimethyl-harnstoff
1-(3,4-Dichlorphenyl)-3-methyl-3-n-butyl-harnstoff
1-(4-i-Propylphenyl)-3,3-dimethyl-harnstoff
1-(3-Trifluormethylphenyl)-3,3-dimethyl-harnstoff
1-(alpha,alpha,beta,beta-Tetrafluorethoxyphenyl)-3,3-dimethyl-harnstoff

1-(3-tert.Butylcarbamoyloxy-phenyl)-3,3-dimethyl-harnstoff
1-(3-Chlor-4-methylphenyl)-3,3-dimethyl-harnstoff
1-(3-Chlor-4-methoxyphenyl)-3,3-dimethyl-harnstoff
1-[4(4'-Chlorphenoxy)-phenyl]-3,3-dimethyl-harnstoff
1-[4(4'-Methoxyphenoxy)-phenyl]-3,3-dimethyl-harnstoff
1-Cyclooctyl-3,3-dimethyl-harnstoff
1-(Hexahydro-4,7-methanindan-5-yl)-3,3-dimethyl-harnstoff
1-[1- oder 2-(3a,4,5,7,7a-Hexahydro)-4,7-methanoindanyl]-3,3-dimethyl-harnstoff
1-(4-Chlorphenyl)-3-methyl-3-methoxy-harnstoff
1-(4-Bromphenyl)-3-methyl-3-methoxy-harnstoff
1-(3,4-Dichlorphenyl)-3-methyl-3-methoxy-harnstoff
1-(3-Chlor-4-bromphenyl)-3-methyl-3-methoxy-harnstoff

1-(2-Benzthiazolyl)-1,3-dimethyl-harnstoff
1-(2-Benzthiazolyl)-3-methyl-harnstoff
1-(5-Trifluormethyl-1,3,4-thiadiazolyl)-1,3-dimethyl-harnstoff
Imidazolidin-2-on-1-carbonsäure-isobutylamid
1,2-Dimethyl-3,5-diphenylpyrazolium-methylsulfat
1,3-Dimethyl-4-(3,4-dichlorbenzoyl)-5-(4-methylphenylsulfonyloxy)-pyrazol
2,3,5-Trichlor-pyridinol-(4)
1-Methyl-3-phenyl-5-(3'-trifluomethylphenyl)-pyridon-(4)
1-Methyl-4-phenyl-pyridiniumchlorid
1,1-Dimethylpyridiniumchlorid
1,1'-Dimethyl-4,4'-dipyridylium-di(methylsulfat)
1,1'-Di(3,5-dimethylmorpholin-carbonylmethyl)-4,4'-dipyridylium-dichlorid
1,1'-Ethylen-2,2'-dipyridylium-dibromid
3-[1-(N-Ethoxyamino)-propyliden]-6-ethyl-3,4-dihydro-2-H-pyran-2,4-dion
3-[1-(N-Allyloxyamino)-propyliden]-6-ethyl-3,4-dihydro-2-H-pyran-2,4-dion
2-[1-(N-Allyloxyamino)-propyliden]-5,5-dimethylcyclohexan-1,3-dion (Salze)
2-[1-(N-Allyloxyamino-butyliden]-5,5-dimethylcyclohexan-1,3-dion (Salze)
2-[1-(N-Allyloxyamino-butyliden]-5,5-dimethyl-4-methoxycarbonyl-cyclohexan-1,3-dion (Salze)
2,4-Dichlorphenoxyessigsäure (Salze, Ester, Amide)
2-Methyl-4-chlorphenoxyessigsäure (Salze, Ester, Amide)
3,5,6-Trichlor-2-pyridinyl-oxyessigsäure (Salze, Ester, Amide)

Alpha-Naphthoxyessigsäuremethylester
2-(2,4-Dichlorphenoxy)-propionsäure (Salze, Ester, Amide)
2-(2-Methyl-4-chlorphenoxy)-propionsäure (Salze, Ester, Amide)
4-(2,4-Dichlorphenoxy)-buttersäure (Salze, Ester, Amide)
4-(2-Methyl-4-chlorphenoxy)-buttersäure (Salze, Ester, Amide)
9-Hydroxyfluoren-carbonsäure-(9) (Salze, Ester)
2,3,6-Trichlorphenyl-essigsäure (Salze, Ester)
4-Chlor-2-oxo-benzothiazolin-3-yl-essigsäure
(Salze, Ester)
Gibellerinsäure (Salze)
Dinatrium-methylarsonat
Mononatriumsalz der Methylarsonsäure
N-Phosphon-methyl-glycin (Salze)
N,N-Bis(phosphonmethyl)-glycin (Salze)
2-Chlorethanphosphonsäure-2-chlorethylester
Ammonium-ethyl-carbamoyl-phosphonat
O,O-Di-n-butyl-(1-n-butylamino-cyclohexyl)-phosphonat
Trithiobutylphosphit

O,O-Diisopropyl-5-(2-benzolsulfonylamino-ethyl)-phosphordithioat
5-tert.Butyl-3-(2,4-dichlor-5-isopropoxyphenyl)-1,3,4-oxadiazolon-(2)
4,5-Dichlor-2-trifluormethyl-benzimidazol (Salze)
1,2,3,6-Tetrahydropyridazin-3,6-dion (Salze)
Bersteinsäure-mono-N,N-dimethylhydrazid (Salze)
(2-Chlorethyl)-trimethyl-ammoniumchlorid
(2-Methyl-4-phenylsulfonyl)-trifluormethansulfonanilid
Ammoniumrhodanid
Calciumcyanamid

2-Chlor-4-trifluormethylphenyl-3'-ethoxycarbonyl-4'-nitrophenylether
1-(4-Benzyloxyphenyl)-3-methyl-3-methoxyharnstoff
3-tert.-Butylamino-4-methoxycarbonyl-5-methylpyrazol
N-Benzyl-N-isopropyl-trimethylacetamid
2-[4-(4'-Chlorphenoxymethyl)-phenoxy]-propionsäuremethylester
2-[4-(5'-Brompyridyl-2-oxy)-phenoxy]-propionsäureethylester
2-[4-(5'-Jodpyridyl-2-oxy)-phenoxy]-propionsäure-n-butylester
2-Chlor-4-trifluormethylphenyl-3'-(2-fluorethoxy)-4'-nitro-phenylether
2-Chlor-4-trifluormethylphenyl-3'-ethoxycarbonylmethylthio-4'-nitrophenylether
2,4,6-Trichlorphenyl-3'-ethoxycarbonyl)methylthio-4'-nitrophenylether
2-[1-(N-ethoxyamino)-butyliden]-5-(2-ethylthiopropyl)-3-hydroxy-cyclohexen-(2)-on-(1) (Salze)
2-[1-(N-ethoxyamino)-butyliden]-5-(2-phenylthiopropyl)-3-hydroxy-cyclohexen-(2)-on-(1) (Salze)

4-[4-(4'-Trifluormethyl)-phenoxy]-penten-2-carbonsäureethylester
2-Chlor-4-trifluormethyl-3'-methoxycarbonyl-4'-nitrophenylether
2,4-Dichlorphenyl-3'-carboxy-4'-nitrophenylether (Salze)
4,5-Dimethoxy-2-(3-alpha,alpha,beta-trifluor-beta-bromethoxyphenyl)-3-(2H)-pyridazinon
2,4-Dichlor-3'[2-(2-ethoxy-ethoxy)-ethoxy]-4'-nitro-diphenyl-ether
2,3-Dihydro-3,3-dimethyl-5-benzofuranyl-ethansulfonat
N-[4-Methoxy-6-methyl-1,3,5-triazin-2-yl-aminocarbonyl]-2-chlorbenzosulfonamid
1-(3-Chlor-4-ethoxyphenyl)-3,3-dimethylharnstoff
2-Methyl-4-chlorphenoxy-thioessigsäureethylester
2-Chlor-3,5-dijod-4-acetoxy-pyridin
1(-4-[2-(4-Methylphenyl)-ethoxy]-phenyl)-3-methyl-3-methoxyharnstoff
2,6-Dimethyl-N-(pyrazolyl-methylenoxymethyl)-2-chloracetanilid
2-Methyl-6-ethyl-N-(pyrazolyl-methylenoxymethyl)-2-chloracetanilid
alpha-2,4-(Dichlorphenoxy-propionsäure)-(3-methoxycarbonylamino)-anilid
1-(alpha-2-Brom-4-chlorphenoxypropionsäure)-3-(0-methylcarbamoyl)-anilid
2-Methyl-6-ethyl-N-(pyrazolyl-ethylenoxymethyl)-2-chloracetanilid

2-(3-Trifluormethylphenyl)-4H-3,1-benzoxazin-4-on
2-(3-Pentafluorethoxyphenyl)-4H-3,1-benzoxazin-4-on
2-(3-Trifluormethylthio-phenyl)-4H-3,1-benzoxazin-4-on
2-(3-Difluorchlormethoxyphenyl)-4H-3,1-benzoxazin-4-on

5-Nitro-2-(3-trifluormethyl-phenyl)-4H-3,1-benzoxazin-4-on
5-Chlor-2-(3-trifluormethoxyphenyl)-4H-3,1-benzoxazin-4-on
5-Chlor-2-(3-alpha,alpha,beta,beta-tetrafluorethoxyphenyl)-4H-3,1-benzoxazin-4-on
5-Fluor-2-(3-alpha,alpha,beta,beta-tetrafluorethoxyphenyl)-4H-3,1-benzoxazin-4-on
5-Chlor-2-(4-difluorchlormethoxyphenyl)-4H-3,1-benzoxazin-4-on
5-Fluor-2-(4-difluorchlormethoxyphenyl)-4H-3,1-benzoxazin-4-on
5-Fluor-2-phenyl-4H-3,1-benzoxazin-4-on
5-Fluor-2-(3-difluormethoxyphenyl)-4H-3,1-benzoxazin-4-on
5-Chlor-2-phenyl-4H-3,1-benzoxazin-4-on
N-3-Chlor-4-isopropylphenyl-thiolcarbaminsäuremethylester
6-Methyl-3-methoxy-5,6-dihydro-1,2,4,6-thiatriazin-5-on-1,1-dioxid Natriumsalz
6-Methyl-3-ethoxy-5,6-dihydro-1,2,4,6-thiatriazin-5-on-1,1-dioxid
5-Amino-4-chlor-2-phenyl-3(2H)-pyridazinon
5-Amino-4-brom-2-phenyl-3(2H)-pyridazinon

5-Methylamino-4-chlor-2-(3-trifluormethylphenyl)-3(2H)-pyridazinon
5-Methylamino-4-chlor-2-(3-alpha,alpha,beta,beta,-tetrafluorethoxyphenyl)-3(2H)-pyridazinon
5-Dimethylamino-4-chlor-2-phenyl-3(2H)-pyridazinon
4,5-Dimethoxy-2-phenyl-3(2H)-pyridazinon
1-[3'-(2"-Chlor-4"-trifluormethylphenoxy)]-phenyl-4,5-dimethoxy-pyridazinon-6
1-[4'-3"-Trifluormethyl-phenoxy)]-phenyl-4,5-dimethoxy-pyridazinon-6
4,5-Dimethoxy-2-(3-alpha,alpha,beta-trifluor-beta-brom-ethoxyphenyl)-3-(2H)-pyridazinon
N-[4-(4'-Methoxy-phenoxy)-3-chlor-phenyl]-carbaminsäuremethylester
N-[4-(4'-Difluormethoxy-phenoxy)-3-chlor-phenyl]-thio-carbaminsäuremethyl-ester
N-[4-(4'-Difluormethoxy-phenoxy)-phenyl]thio-carbaminsäuremethylester
1-[4-(4'-Methylphenylpropyl)-phenyl]-3-methyl-3-methoxyharnstoff
1-[3-(4'-Chlorphenyl-propyl)-phenyl]-3-methyl-3-methoxyharnstoff
1-[4-(3-Phenyl-2-methyl-propyl)-phenyl]-3-methyl-methoxyharnstoff
1-[4-(3-)4'-Chlorphenyl)-2-methyl-propyl)-phenyl]-3-methyl-3-methoxyharnstoff
1-[4-(3-(4'-Methylphenyl)-2-methylpropyl)-phenyl]-3-methyl-3-methoxyharnstoff
2-[1-(N-Ethyloxyamino)-butyliden]-5(4-ethylphenyl)-3-hydroxy-cyclohexen-(2)-on-(1) (Salze)
2-[1-(N-Ethyloxyamino)-butyliden]-5(4-fluorphenyl)-3-hydroxy-cyclohexen-(2)-on-(1) (Salze)
2-[1-(N-Ethyloxyamino)-butyliden]-5-(4-chlorphenyl)-3-hydroxy-cyclohexen-(2)-on-(1) (Salze)
2'-(2,4,6-Trichlorphenyl)-hydrazino-2-cyanacrylsäuremethylester
2-[1-(N-Ethyloxamino)-butyliden]-5-(1,3,3-trimethyl-cyclohexen-1-yl-2)-3-hydroxy-cyclohexen-(2)-on-(1) (Salze)
2-[1-(N-Ethyloxamino)-butyliden]-5-(2,4,4-trimethyl-cyclohexen-1-yl-3)-3-hydroxy-cyclohexen-(2)-on-(1) (Salze)
2-[1-(N-3-Chlorallyl-oxamino-butyliden]-5-(1-methyl-cyclohex-1-en-4-yl)-3-hydroxy-cyclohexen-(2)-on-(1) (Salze)
3-Ixobutoxy-5-methyl-4-methoxycarbonyl-pyrazol
5-Amino-1-(2,4,6-trichlorphenyl)-4-cyano-pyrazol
5-Amino-1-(2,4,6-tribromphenyl)-4-cyano-pyrazol

5-Amino-1-(2,4,6-trichlorphenyl)-4-methoxycarbonyl-pyrazol
5-Amino-(2,4-dichlor-6-bromphenyl)-4-methoxycarbonyl-pyrazol
5-Amino-(2,6-dichlor-4-bromphenyl)-4-methoxycarbonyl-pyrazol
5-Chlor-2-(3-trifluormethyl-phenyl)-4H-3,1-benzoxazin-4-on
5-Fluor-2-(3-trifluormethyl-phenyl)-4H-3,1-benzoxazin-4-on
2-(3-Tetrafluorethoxyphenyl)-4H-3,1-benzoxazin-4-on
5-Chlor-2-(4'-fluorphenyl)-4H-3,1-benzoxazin-4-on
5-Fluor-2-(4'-fluorphenyl)-4H-3,1-benzoxazin-4-on
5-Fluor-2-(3'-fluorphenyl)-4H-3,1-benzoxazin-4-on
5-Chlor-2-(3'-fluorphenyl)-4H-3,1-benzoxazin-4-on
5-Chlor-2-(3'-difluorchlormethylphenyl)-4H-3,1-benzoxazin-4-on
5-Fluor-2-(3'-difluorchlormethylphenyl)-4H-3,1-benzoxazin-4-on

6-Methyl-3-methoxy-5-(4'-nitrophenoxy)-6H-1,2,4,6-thiatriazin-1,1-dioxid
6-Methyl-3-methoxy-5-(propargyloxy)-6H-1,2,4,6-thiatriazin-1,1-dioxid
6-Methyl-3-methoxy-5-(2,4-dichlorbenzoxy)-6H-1,2,4,6-thiatriazin-1,1-dioxid
2-(2',4'-Dichlorphenoxy)-2-fluorpropionsäure (Salze, Ester)
2-[4-(5'-Trifluormethylpyridyl-2-oxy)-phenoxy]-propionsäurebutylester
2-[4-(3'-Chlor-5'-trifluormethylpyridyl-2-oxy)-phenoxy]-propionsäure (Salze, Ester)
2-[4-(6-Chlorchinoxalyl-2-oxy)-phenoxy]-propionsäure-pentylester
2-[4-(6-Chlor-chinoxalyl-2-oxy)-phenoxy]-propionsäuremethylester
2-[4-(6-Chlorbenzthiazolyl-2-oxy)-phenoxy]-propionsäure (Salze, Ester)
2-[4-(6-Chlorbenzoxazolyl-2-oxy)-phenoxy]-propionsäure (Salze, Ester)
1-[5-(3-Fluorbenzylthio)-thiadiazolyl-2]-1-methylharnstoff
2-Methoxycarbonyl-N-(3,5-dimethylpyrimidinyl-2-aminocarbonyl)-benzolsulfonamid
alpha-(3,5,6-Trichlor-pyrid-2-yl-oxy)-essigsäure (Salze, Ester)
alpha-(4-Amino-3,5-dichlor-6-fluor-pyrid-2-yl-oxy)-essigsäure (Salze, Ester)
S-[N-(4-Chlorphenyl)-N-isopropyl-carbamoyl-methyl]-O,O-dimethyl-dithiophosphat
Ammonium-(3-amino-3-carboxy-propyl)-methylphosphinat
(Hydroxy)-(methyl)-phophinyl-L-alpha-aminobutyryl-L-alanyl-Natriulsalz
4-Trifluormethyl-diphenylether
2-(3,5-Dichlorphenyl)-2-(2'2'2'-trichlorethyl)-oxiran
2,4-Diamino-5-methylthio-5-chlor-pyrimidin
N-(4-Ethylthio-2-trifluormethyl-phenyl)-methylsulfonamid
3-Methoxy-4-methyl-5(3-methyl-2-butenyloxy)-1,2-di(hydroxymethyl)-benzol
2-(3,5-Dimethylphenoxy)-2-(1,2,4-triazolyl-1)-essigsäure-N-tertiär-butylamid
2-(3,5-Dichlorphenoxy)-2-(1,2,4-triazolyl-1)-essigsäure-N-tertiär-butylamid
3,7-Dichlor-8-chinolincarbonsäure (Salze, Ester)
5-[2-Chlor-4-trifluormethyl-phenoxy-N-1-(methoxycarbonyl)-ethoxy]-2-nitrobenzamid
N-[3-(1-Ethyl-1-methylpropyl)-isoxazolyl-5]-2,6-dimethoxybenzamid
2'-Methoxyethyl-2-[5-(2-chlor-4-trifluormethyl-phenoxy)-2-nitrophenoxy]-propionat
Methyl-6-(4-isopropyl-4-methyl-5-oxo-2-imidazolin-2-yl)-3-methylbenzoat
Methyl-6-(4-isopropyl-4-methyl-5-oxo-2-imidozin-2-yl)-4-methylbenzoat
Benzyltrimethylammoniumchlorid

1-[alpha-(4-Trifluormethyl-phenoxy)-phenoxy-propionsäure]-3-(O-methyl-carbamoyl)-anilid
1-Dodecyl-cycloheptan-2-on
N-[2-Chlor-4-methylsulfonyl-phenyl]-chlormethansulfonamid
N-[2-Brom-4-ethylsulfonyl-phenyl]-chlormethansulfonamid
N-[2,3-Dichlor-4-ethylsulfonyl-phenyl]-chlormethansulfonamid
2-[1-(N-Ethoxyamino)-pyroppyliden-]-5-(pyrid-3-yl)-3-hydroxy-cyclohex-2-en-1-on (Salze)
2,4,5-Trichlorphenoxyessigsäure (Salze, Ester, Amide)

2-[1-(N-Ethoxyamino)-butyliden]-5-(tetrahydropyran-3-yl)-3-hydroxy-cyclohex-2-en-1-on (Salze)
2-[1-(N-Ethoxyamino)-butyliden]-5-(4-methyl-tetrahydropyran-3-yl)-3-hydroxy-cyclohex-2-en-1-on (Salze)
2-[1-(N-Ethoxyamino)-butyliden]-5-(tetrahydrothiopyran-3-yl)-3-hydroxy-cyclohex-2-en-1-on (Salze)
2-[1-(N-Ethoxyamino)-propyliden]-5-(pyrid-3-yl)-3-hydroxy-cyclohex-2-en-1-on (Salze)
2-[1-(N-Allyloxamino)-propyliden]-5-(pyrid-3-yl)-3-hydroxy-cyclohex-2-en-1-on
2-[1-(N-Ethoxyamino)-butyliden]-5-(pyrid-3-yl)-3-hydroxy-cyclohex-2-en-1-on (Salze)
2-[1-(N-Allyloxyamino)-butyliden]-5-(pyrid-3-yl)-3-hydroxy-cyclohex-2-en-1-on (Salze)

2-[4,5-Dihydro-4-methyl-4-isopropyl-5-oxo-1H-imidazol-2-yl]-3-chinolin-carbonsäure
2-[4,5-Dihydro-4-methyl-4-isopropyl-5-oxo-1H-imidazol-2-yl]-nicotinsäure-isopropylaminsalz
2-Chlor-2'-methyl-6'-ethyl-N-(N'-1-methoxycarbonyl-ureidomethylacetanilid

2-Chlor-2'-6'-diethyl-N-(N'-1-methoxycarbonyl)-ureidomethylacetanilid
2-Chlor-2'-6'-dimethyl-N-(N'-1-methoxycarbonyl)-ureidomethylacetanilid
2-Chlor-6-nitro-3-phenoxy-anilin
N-Phosphonomethyl-glycin-trimethyl-sulfoniumsalz
5-(2-Chlor-4-trifluormethyl-phenoxy)-2-nitro-N-methansulfonyl-benzamid
5-(2-Chlor-4-trifluormethyl-phenoxy)-2-nitro-benzoesäure-1-ethoxycarbonylethyl)-ester
1-[3'-(2''-Chlor-4''-trifluormethyl-phenyl-thio)-phenyl]-4,5-dimethoxy-pyridazon-(6)
3-Methyl-6-fluor-5H-thiazolo[2,3-b]-chinazolin-5-on
3-Methyl-2-sulfosäure-5H-thiazolo[2,3-b]-chinazolin-5-on
3-Methyl-2-brom-5H-thiazolo[2,3-b]-chinazolin-5-on
5H-Triazolo[2,3-b]-chinazolin-5-on
2-(1-Ethoxyamino-butyliden)-5-cyclododeca-1,5-dion-9-yl-cyclohex-1-en-1-on
2-(1-Ethoxyamino-butyliden)-5-cyclododecyl-cyclohex-1-en-1-on
5-(4-Trifluormethyl-2-chlor-phenoxy)-2-nitro-benzylthioessigsäure-(2-trimethylsilylethyl)-ester
2-(2-Chlorbenzyl)-4,4-dimethyl-isoxazolidin-3-on

Außerdem kann es von Nutzen sein, die Verbindungen der Formel I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Bekämpfung von Ernährungs- und Spurenelementmängeln eingesetzt werden. Es können auch nichtphytotoxische Öle und Ölkonzentrate zugesetzt werden.

**Patentansprüche**

1. Cyclohexan-1,3-dionderivate der Formel

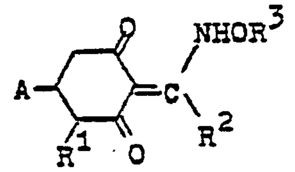

(I)

in der

A einen 4- bis 7-gliedrigen, gegebenenfalls ungesättigten Ring, der 1 oder 2 Sulfinyl- oder Sulfonylgruppen enthält und gegebenenfalls durch maximal 3 Alkylgruppen mit jeweils bis zu 4 C-Atomen, 2 Alkenylgruppen mit jeweils bis zu 6 C-Atomen oder 3 Alkoxygruppen mit jeweils bis zu 2 C-Atomen substituiert sein kann,

$R^1$ Wasserstoff, Methoxycarbonyl,

$R^2$ Alkyl mit 1 bis 4 C-Atomen und

$R^3$ Alkyl mit 1 bis 4 C-Atomen, alkenyl mit 3 oder 4 C-Atomen, Halogenalkenyl mit 3 oder 4 C-Atomen und 1 bis 3 Halogensubstituenten oder Propargyl bedeuten,

und Salze dieser Verbindungen.

2. Cyclohexan-1,3-dionderivate der Formel I gemäß Anspruch 1, dadurch gekennzeichnet, daß $R^1$ Wasserstoff bedeutet.

3. Cyclohexan-1,3-dionderivate der Formel I gemäß Anspruch 1, dadurch gekennzeichnet, daß A einen 5- oder 6-gliedrigen, gegebenenfalls ungesättigten Ring, der 1 oder 2 Sulfinyl- oder Sulfonylgruppen enthält und gegebenenfalls durch maximal 3 Alkylgruppen mit jeweils bis zu 4 C-Atomen, 2 Alkenylgruppen mit jeweils bis zu 6 C-Atomen oder 3 Alkoxygruppen mit jeweils bis zu 2 C-Atomen substituiert sein kann, bedeutet.

4. Verfahren zur Herstellung eines Cyclohexan-1,3-dionderivates der Formel I gemäß Anspruch 1, dadurch gekennzeichnet, daß man eine Verbindung der Formel

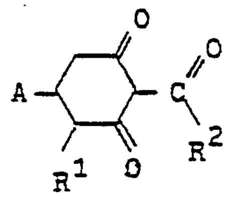

(II)

in der A, $R^1$ und $R^2$ die im Anspruch 1 genannten Bedeutungen haben,

a) mit einer Ammoniumverbindung der Formel $R^3O—NH_3Y$, in der $R^3$ die im Anspruch 1 genannten Bedeutungen hat und Y ein Anion bedeutet, in einem inerten Verdünnungsmittel bei einer Temperatur zwischen 0 und 80 °C in Gegenwart einer Base oder

b) mit einem gegebenenfalls in wäßriger Lösung vorliegenden Hydroxylamin der Formel $R^3O—NH_2$, in der $R^3$ die im Anspruch 1 genannten Bedeutungen hat, in einem inerten Lösungsmittel bei einer Temperatur zwischen 0 °C und dem Siedepunkt des Reaktionsgemisches umsetzt.

5. Verfahren zur Herstellung eines Cyclohexan-1,3-dionderivates der Formel I gemäß Anspruch 1, dadurch gekennzeichnet, daß man ein entsprechendes Cyclohexan-1,3-dionderivat, in dem der Schwefel eine niedere Oxidationsstufe besitzt, in an sich üblicher Weise zum Sulfoxid oder Sulfon der allgemeinen Formel I gemäß Anspruch 1 oxidiert.

6. Herbizid, enthaltend ein Cyclohexan-1,3-dionderivat der Formel I gemäß Anspruch 1 oder Salze dieser Verbindung.

7. Herbizid, enthaltend inerte Zusatzstoffe und ein Cyclohexan-1,3-dionderivat der Formel

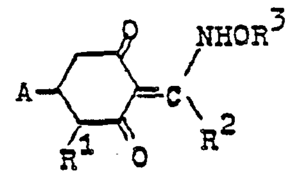

(I)

in der

A einen 4- bis 7-gliedrigen, gegebenenfalls ungesättigten Ring, der 1 oder 2 Sulfinyl- oder Sulfonylgruppen enthält und gegebenenfalls durch maximal 3 Alkylgruppen mit jeweils bis zu 4 C-Atomen, 2 Alkenylgruppen mit jeweils bis zu 6 C-Atomen oder 3 Alkoxygruppen mit jeweils bis zu 2 C-Atomen substituiert sein kann,

$R^1$ Wasserstoff, Methoxycarbonyl,

$R^2$ Alkyl mit 1 bis 4 C-Atomen und

$R^3$ Alkyl mit 1 bis 4 C-Atomen, Alkenyl mit 3 oder 4 C-Atomen, Halogenalkenyl mit 3 oder 4 C-Atomen und 1 bis 3 Halogensubstituenten oder Propargyl bedeuten,

oder Salze dieser Verbindung.

8. Herbizid nach Anspruch 6, dadurch gekennzeichnet, daß es ein Cyclohexan-1,3dionderivat der Formel I enthält, wobei $R^1$ Wasserstoff bedeutet.

9. Herbizid nach Anspruch 6, dadurch gekennzeichnet, daß es ein Cyclohexan-1,3-dionderivat der Formel I enthält, wobei A einen 5- oder 6-gliedrigen, gegebenenfalls ungesättigen Ring, der 1 oder 2 Sulfinyl- oder Sulfonylgruppen enthält und gegebenenfalls durch maximal 3 Alkylgruppen mit jeweils bis zu 4 C-Atomen, 2-Alkenylgruppen mit jeweils bis zu 6 C-Atomen oder 3 Alkoxygruppen mit jeweils bis zu 2 C-Atomen substituiert sein kann, bedeutet.

10. Verfahren zur Bekämpfung unerwünschten Pflanzenwachstums, dadurch gekennzeichnet, daß man die unerwünschten Pflanzen und/oder die von unerwünschten Pflanzenwachstum freizuhaltende Fläche mit einer herbizid wirksamen Menge eines Cyclohexan-1,3-dionderivates der Formel I gemäß Anspruch 1 oder Salzen dieser Verbindung behandelt.

**Claims**

1. A cyclohexane-1,3-dione derivative of the formula

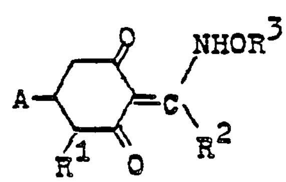

(I)

where

A is a saturated or unsaturated 4-membered to 7-membered ring which contains 1 or 2 sulfinyl or sulfonyl groups and is unsubstituted or substituted by not more than 3 alkyl groups, each of not more than 4 carbon atoms, 2 alkenyl groups, each of not more than 6 carbon atoms, or 3 alkoxy groups, each of not more than 2 carbon atoms,

$R^1$ is hydrogen or methoxycarbonyl,

$R^2$ is alkyl of 1 to 4 carbon atoms, and

$R^3$ is alkyl of 1 to 4 carbon atoms, alkenyl of 3 or 4 carbon atoms or haloalkenyl of 3 or 4 carbon atoms which has 1 to 3 halogen substituents, or is propargyl,

or a salt thereof.

2. A cyclohexane-1,3-dione derivative of the formula I as claimed in claim 1, where $R^1$ is hydrogen.

3. A cyclohexane-1,3-dione derivative of the formula I as claimed in claim 1, where A is a saturated or unsaturated, 5- or 6-membered ring which contains 1 or 2 sulfinyl or sulfonyl groups and is unsubstituted or substituted by not more than 3 alkyl groups, each of not more than 4 carbon atoms, 2 alkenyl groups, each of not more than 6 carbon atoms, or 3 alkoxy groups, each of not more than 2 carbon atoms.

4. A process for the preparation of a cyclohexane-1,3-dione derivative of the formula I as claimed in claim 1, wherein a compound of the formula

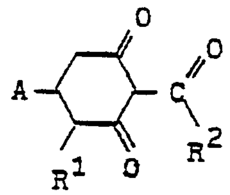
(II)

where A, $R^1$ and $R^2$ have the meanings given in claim 1, is reacted

a) with an ammonium compound of the formula $R^3O—NH_3Y$, where $R^3$ has the meanings given in claim 1, and Y is an anion, in an inert diluent at from 0 to 80 °C in the presence of a base, or

b) with a hydroxylamine which may, if desired, be in the form of an aqueous solution and is of the formula $R^3O—NH_2$, where $R^3$ has the meanings given in claim 1, in an inert solvent at from 0 °C to the boiling point of the reaction mixture.

5. A process for the preparation of a cyclohexane-1,3-dione derivative of the formula I as claimed in claim 1, wherein an appropriate cyclohexane-1,3-dione derivative in which the sulfur has a lower oxidation state is oxidized in a conventional manner to the sulfoxide or sulfone of the general formula I as claimed in claim 1.

6. A herbicide containing a cyclohexane-1,3-dione derivative of the formula I as claimed in claim 1, or a salt thereof.

7. A herbicide containing inert additives and cyclohexane-1,3-dione derivative of the formula

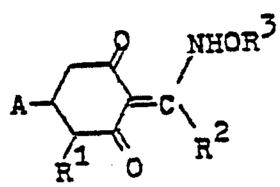
(I)

where

A is a saturated or unsaturated 4-membered to 7-membered ring which contains 1 or 2 sulfinyl or sulfonyl groups and is unsubstituted or substituted by not more than 3 alkyl groups, each of not more than 4 carbon atoms, 2 alkenyl groups, each of not more than 6 carbon atoms, or 3 alkoxy groups, each of not more than 2 carbon atoms,

$R^1$ is hydrogen or methoxycarbonyl

$R^2$ is alkyl of 1 to 4 carbon atoms, and

$R^3$ is alkyl of 1 to 4 carbon atoms, alkenyl of 3 or 4 carbon atoms or haloalkenyl of 3 or 4 carbon atoms which has 1 to 3 halogen substituents, or is propargyl,

or a salt thereof.

8. A herbicide as claimed in claim 6, containing a cyclohexane-1,3-dione derivative of the formula I where $R^1$ is hydrogen.

9. A herbicide as claimed in claim 6, containing a cyclohexane-1,3-dione derivative of the formula I where A is a saturated or unsaturated, 5- or 6-membered ring which contains 1 or 2 sulfinyl or sulfonyl groups and is unsubstituted or substituted by not more than 3 alkyl groups, each of not more than 4 carbon atoms, 2 alkenyl groups, each of not more than 6 carbon atoms, or 3 alkoxy groups, each of not more than 2 carbon atoms.

10. A process for combating the growth of unwanted plants, wherein the unwanted plants and/or the area to be kept free from unwanted plan growth are treated with a herbicidally effective amount of a cyclohexane-1,3-dione derivative of the formula I as claimed in claim 1, or a salt thereof.

**Revendications**

1. Dérivés de cyclohexane-1,3-dione de formule

(voir dessin page 26)

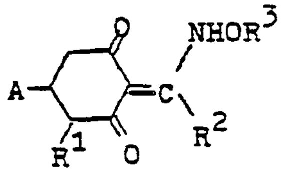

(I)

dans laquelle

A représente un noyau de 4 à 7 chaînons, éventuellement insaturé, qui contient 1 ou 2 groupes sulfinyle ou sulfonyle et qui peut éventuellement être substitué par, au maximum, 3 groupes alkyle ayant chacun jusqu'à 4 atomes C, 2 groupes alcényle ayant chacun jusqu'à 6 atomes C ou 3 groupes alcoxy ayant chacun jusqu'à 2 atomes C,

$R^1$ représente hydrogène, méthoxycarbonyle,

$R^2$ alkyle ayant 1 à 4 atomes C et

$R^3$ alkyle ayant 1 à 4 atomes C, alcényle à 3 ou 4 atomes C, halogénalkyle à 3 ou 4 atomes C et 1 à 3 substituants halogène ou propargyle,

et sels de ces composés.

2. Dérivés de cyclohexane-1,3-dione de formule I selon la revendication 1, caractérisé par le fait que $R^1$ représente hydrogène.

3. Dérivés de cyclohexane-1,3-dione de formule 1 selon la revendication 1, caractérisé par le fait que A représente un noyau à 5 ou 6 chaînons, éventuellement insaturé, qui contient 1 ou 2 groupes sulfinyle ou sulfonyle et qui peut éventuellement être substitué par, au maximum, 3 groupes alkyle ayant chacun jusqu'à 4 atomes C, 2 groupes alcényle ayant chacun jusqu'à 6 atomes C ou 3 groupes alcoxy ayant chacun jusqu'à 2 atomes C.

4. Procédé de préparation d'un dérivé de cyclohexane-1,3-dione de formule I selon la revendication 1, caractérisé par le fait que l'on fait réagir un composé de formule

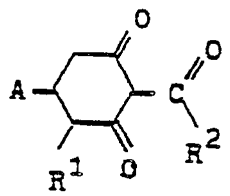

(II)

dans laquelle A, $R^1$ et $R^2$ ont les significations données dans la revendication 1,

a) avec un composé d'ammonium de formule $R^3O—NH_3Y$, dans laquelle $R^3$ a la signification donnée dans la revendication 1 et Y représente un anion, dans un diluant inerte, à une température comprise entre 0 et 80 °C, en présence d'une base, ou

b) avec une hydroxylamine de formule $R^3O—NH_2$, se trouvant éventuellement en solution aqueuse, $R^3$ ayant la signification donnée dans la revendication 1, dans un solvant inerte, à une température comprise entre 0 °C et le point d'ébullition du mélange de réaction.

5. Procédé de préparation d'un dérivé de cyclohexane-1,3-dione de formule I selon la revendication 1, caractérisé par le fait que l'on oxyde, de manière usuelle, en sulfoxyde ou sulfone de la formule générale I selon la revendication 1, un dérivé de cyclohexane-1,3-dione correspondant, dans lequel le soufre possède un stade d'oxydation inférieur.

6. Herbicide contenant un dérivé de cyclohexane-1,3-dione de formule I selon la revendication 1 ou des sels de ce composé.

7. Herbicide contenant des additifs inertes et un dérivé de cyclohexane-1,3-dione de formule

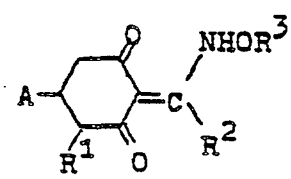

(I)

dans laquelle

A représente un noyau de 4 à 7 chaînons, éventuellement insaturé, qui contient 1 ou 2 groupes sulfinyle ou sulfonyle et qui peut éventuellement être substitué par, au maximum, 3 groupes alkyle ayant chacun jusqu'à 4 atomes, 2 groupes alcényle ayant chacun jusqu'à 6 atomes C ou 3 groupes alcoxy ayant chacun jusqu'à 2 atomes C,

$R^1$ représente hydrogène, méthoxycarbonyle,

$R^2$ alkyle ayant 1 à 4 atomes C et

$R^3$ alkyle ayant 1 à 4 atomes C, alcényle à 3 ou 4 atomes C, halogénalkyle à 3 ou 4 atomes C et 1 à 3 substituants halogène ou propargyle,
et sels de ces composés.

8. Herbicide selon la revendication 6, caractérisé par le fait qu'il contient un dérivé de cyclohexane-1,3-dione de formule I selon la revendication 1, $R^1$ représentant hydrogène.

9. Herbicide selon la revendication 6, caractérisé par le fait qu'il contient un dérivé de cyclohexanne-1,3-dione de formule I selon la revendication 1, A représentant un noyau à 5 ou 6 chaînons, éventuellement insaturé, qui contient 1 ou 2 groupes sulfinyle ou sulfonyle et qui peut éventuellement être substitué par, au maximum, 3 groupes alkyle ayant chacun jusqu'à 4 atomes C, 2 groupes alcényle ayant chacun jusqu'à 6 atomes C ou 3 groupes alcoxy ayant chacun jusqu'à 2 atomes C.

10. Procédé de lutte contre une croissance des plantes indésirables, caractérisé par le fait que l'on traite les plantes indésirables et/ou les surfaces à maintenir exemptes d'une croissance indésirable des plantes, avec une quantité efficace au point de vue herbicide d'un dérivé de cyclohexane-1,3-dione de formule I selon la revendication 1 ou des sels de ce composé.